**Europäisches Patentamt**

**European Patent Office**

**Office européen des brevets**

(11) Numéro de publication: **0 254 607 B1**

(12) **FASCICULE DE BREVET EUROPEEN**

(45) Date de publication de fascicule du brevet: **01.04.92** (51) Int. Cl.⁵: **A61M 1/02**

(21) Numéro de dépôt: **87401442.6**

(22) Date de dépôt: **23.06.87**

(54) **Dispositif d'autotransfusion sanguine.**

(30) Priorité: **24.06.86 FR 8609117**

(43) Date de publication de la demande:
**27.01.88 Bulletin 88/04**

(45) Mention de la délivrance du brevet:
**01.04.92 Bulletin 92/14**

(84) Etats contractants désignés:
**AT BE CH DE ES GB GR IT LI LU NL SE**

(56) Documents cités:
**US-A- 4 006 745**

(73) Titulaire: **FUTUR-OUOTIDIEN S.A. Société dite
:
251 avenue de la Marne
F-33700 Merignac(FR)**

(72) Inventeur: **Lasnier, Evelyne
18 rue E. Goncourt
F-94000 Creteil(FR)**
Inventeur: **Lasnier, Hubert
18 rue E. Goncourt
F-94000 Creteil(FR)**
Inventeur: **Perovitch, Philippe
251 avenue de la Marne
F-33700 Merignac(FR)**

(74) Mandataire: **Wagret, Jean-Michel et al
CABINET WAGRET 23, rue de Léningrad
F-75008 Paris(FR)**

Rank Xerox (UK) Business Services

## Description

La présente invention est relative à un dispositif de récupération et réinjection de sang, plus particulièrement destiné à compenser les hémorragies de malades ou blessés graves dans des situations d'interventions chirurgicales graves ou longues ou en cas de conditions chirurgicales difficiles, à savoir dans des circonstances où il est impossible de disposer rapidement de sang du même groupe pour réaliser une transfusion efficace.

Actuellement, en prévision d'une intervention chirurgicale particulièrement sanglante, les Anesthésistes-Réanimateurs commandent à l'avance les poches de sang,dont ils ont besoin, aux Centres de Transfusion Sanguine (C.T.S.).

Toutefois, en raison du coût relativement élevé du sang mis à disposition par les C.T.S. et de sa durée de validité relativement réduite, les Centres d'Intervention Chirurgicale ne peuvent pas se permettre un stockage important desdites poches de sang, dont l'approvisionnement se fait au jour le jour, en fonction des prévisions, ce qui ne permet pas de faire face, d'une part, aux situations d'interventions chirurgicales graves, dans lesquelles se produit une hémorragie grave et importante par rupture accidentelle d'une grosse veine ou d'une grosse artère et, d'autre part, aux situations d'interventions chirurgicales dont la durée est supérieure à ce qu'on avait pu prévoir, ce qui est par exemple le cas de la Chirurgie Lourde Cardiovasculaire.

En outre, dans le cas d'interventions chirurgicales effectuées en conditions difficiles, notamment en cas de catastrophes telles que tremblements de terre, accidents ferroviaires, catastrophes industrielles, etc..., on se heurte à des défauts d'approvisionnement de grandes quantités de sang pour transfusion dont on a besoin dans de telles circonstances pour soigner les blessés hémorragiques.

Les problèmes qui se présentent dans le cas de catastrophes se posent également dans le cas de la chirurgie des blessés de guerre (qui se déroule toujours sur ou à proximité du champ de bataille) à cause des difficultés d'approvisionnement, en qualité et en quantité de sang, nécessaire pour les transfusions.

La présente invention a en conséquence pour but de pourvoir à un dispositif de récupération et réinjection de sang destiné à éliminer les inconvénients susdits et, d'une façon générale, à permettre de restituer immédiatement à un patient ou blessé perdant son sang, une partie de son hémorragie, et ce dans de bonnes conditions de sécurité.

De façon plus précise, le dispositif selon l'invention est avantageux en ce que :

- en situation d'intervention chirurgicale grave et/ou longue, le dispositif assure une sécurité supplémentaire permettant de récupérer et restituer le sang du patient, dont la circulation sanguine peut être ainsi maintenue dans un état satisfaisant en attendant la fourniture des volumes de sang transfusionnel nécessaires à une telle situation d'urgence, ceci sur un plan quantitatif ;

- en situation d'intervention chirurgicale difficile, le dispositif permet de pallier en permanence auxdits défauts d'approvisionnement de sang et d'améliorer les conditions et le pronostic de ce type d'interventions chirurgicales, en particulier en raison du fait que dans la plupart des cas, ces situations ne permettent pas non plus de disposer de quantités de sang transfusionnel correspondant au groupe sanguin des blessés ;

- dans chacune des situations susdites, il n'y a pas de risques d'incompatibilité transfusionnelle étant donné que le patient est transfusé avec son propre sang ;

- en dehors des situations d'urgence susdites, le dispositif peut être utilisé systématiquement, d'une part, pour diminuer le coût des interventions chirurgicales normales, étant donné que les transfusions sanguines coûtent cher, et ce en récupérant le sang d'un patient qui, outre à gêner l'intervention du chirurgien par son écoulement dans le champ opératoire, représente une perte économique importante (à l'heure actuelle, ce sang est notoirement aspiré dans des conditions stériles, stocké dans un bocal et puis jeté) et, d'autre part, pour réduire les risques de transmission de maladies virales ou de réactions immunologiques que présentent les transfusions classiques, dites hétérotransfusions isogroupe, ce qui est particulièrement avantageux notamment dans le cas de maladies comme le SIDA, ou l'Hépatite Virale, dont la transmission par des transfusions de sang a été désormais démontrée ;

- le dispositif permet de restituer au malade hémorragique ses propres cellules de défense (globules blancs, lymphocytes), c'est-à-dire sa "mémoire" antigénique, ses anticorps circulants, ses plaquettes et ses protéines sériques, évitant ainsi d'utiliser par trop des substances inertes dites de remplissage ;

- le dispositif permet de secourir utilement certains patients ou blessés dont les convictions religieuses interdisent transfusions, greffes d'organes et produits biologiques ou de synthèse fabriqués (notoirement les Témoins de Jehovah et les Mormons) ;

- le dispositif peut apporter une sécurité supplémentaire lors des interventions chirurgicales, et ce précisément grâce à la récupération de sang qu'il permet d'effectuer, dans un grand nombre de pays qui - tout en possédant des installations chirurgicales de très bon niveau et des chirurgiens compétents - ne disposent pas de Centre de Transfusion Sanguine (c'est le cas de la Côte d'Ivoire, par exemple) ;

- le dispositif permet le stockage au froid du sang récupéré sur un malade, lorsque l'on souhaite lui faire bénéficier de cette transfusion à distance du prélèvement, et
- le dispositif est léger et son encombrement très réduit, donc facilement maniable.

La présente invention a pour objet un dispositif de récupération et de réinjection de sang, plus particulièrement en vue de la réinjection dans l'organisme du sujet de son propre sang en vue de compenser une hémorragie, du type comportant des moyens stériles de récupération du sang par aspiration et aptes à être positionnés à proximité immédiate du point de rupture des vaisseaux, des moyens de filtration en vue d'éliminer les plaquettes sanguines coagulées, des moyens d'injection dans le sang recueilli de produits anti-coagulants et des moyens de mise en circulation du sang permettant d'une part son aspiration et d'autre part sa réinjection vers une tubulure reliée à des moyens de transfusion dans le système circulatoire sanguin du sujet, caractérisé en ce que les moyens de prélèvement et de récupération par aspiration sont reliés par une tubulure à une première enceinte de filtration apte à recueillir le sang, l'enceinte étant pourvue de lits filtrants superposés, sous vide, la base de l'enceinte de filtration étant à cet effet apte à être mise en communication avec une source de dépression, et ladite enceinte de filtration est en communication à son sommet avec une source de produits anti-coagulants, permettant la dispersion desdits produits anti-coagulants sous forme de brouillard apte à remplir la phase gazeuse de ladite enceinte de filtration au-dessus et en amont des lits filtrants, l'enceinte de filtration étant en communication par sa base avec une seconde enceinte ou enceinte de collecte et de stockage située en-dessous de l'enceinte de filtration, l'enceinte de collecte de stockage étant ainsi apte à recueillir le sang s'écoulant par gravité depuis l'enceinte de filtration, ladite enceinte de collecte et de stockage comportant une seconde réserve d'agents anti-coagulants en communication avec ladite enceinte de collecte et de stockage par des moyens de dispersion permettant la diffusion desdits agents dans le sang en cours de stockage dans ladite enceinte, la base de l'enceinte de collecte et de stockage communiquant par un tube de sortie inférieur avec des moyens de rétrotransfusion dans le système circulatoire sanguin du sujet par des seconds moyens de filtration.

Les moyens de mise en circulation du sang sont avantageusement constitués d'une part de moyens d'aspiration notamment d'une source de dépression et d'autre part de moyens de refoulement comportant notamment au moins une source de surpression et le dispositif comporte une vanne à plusieurs voies apte à assurer alternativement la connection de l'ensemble des deux enceintes respectivement de filtration et de collecte avec la source de dépression en vue de la récupération du sang ou la connection de l'enceinte de collecte avec les moyens de refoulement tels qu'une source de surpression en vue de la réinjection du sang dans le système circulatoire du sujet.

Plus particulièrement l'enceinte de collecte et de stockage communique par son sommet d'une part avec la source de dépression et d'autre part avec l'enceinte de filtration, la dépression ainsi engendrée au sein de l'enceinte de stockage se communiquant à l'enceinte de filtration et permettant l'aspiration et la filtration sous vide partiel du sang et son écoulement depuis l'enceinte de filtration vers l'enceinte de collecte, et ladite enceinte de collecte et de stockage communique également par son sommet avec une source de surpression apte à faciliter par surpression dans la phase gazeuse de ladite enceinte de collecte et de stockage la mise en circulation du sang vers les moyens de réinjection par transfusion dans le système sanguin du sujet, la communication entre les deux enceintes (enceinte de filtration et enceinte de stockage) entre elles d'une part et la communication de l'enceinte de stockage avec la source de dépression ou la source de surpression d'autre part étant commandée par une vanne unique à plusieurs voies, ladite vanne étant apte pour une première position à isoler l'enceinte de stockage de la source de surpression en mettant cette enceinte en communication avec la source de dépression et avec l'enceinte de filtration, position pour laquelle le sang est aspiré vers l'enceinte de filtration et au-delà vers l'enceinte de stockage, la vanne étant apte, pour une seconde position, à isoler l'enceinte de stockage par rapport à la source de dépression et par rapport à l'enceinte de filtration, en mettant ladite enceinte de stockage en communication avec la source de surpression, position pour laquelle la surpression engendrée dans l'enceinte de stockage facilite le déplacement du sang vers les moyens de transfusion.

Selon une forme de réalisation l'enceinte de collecte et de stockage est constituée d'une poche en matière plastique souple apte à recevoir le sang provenant de l'enceinte de filtration, cette poche communiquant en sa partie supérieure et par ladite vanne avec l'enceinte de filtration et par sa base avec un circuit d'évacuation comportant notamment des seconds moyens de filtration, ladite enceinte de stockage étant positionnée à l'intérieur d'une cuve rigide, l'espace entre la paroi de la cuve et ladite enceinte ou poche formant une chemise d'air, ladite chemise d'air étant en communication par un passage formant reniflard avec ladite poche et étant mise en communication par son sommet et par l'intermédiaire de la vanne à plusieurs voies alternativement avec la source de dépression ou la source de surpression suivant le positionnement de ladite vanne.

De préférence la cuve de filtration a une forme générale tronconique, dont la petite base est en position inférieure, et comporte un fond en forme d'entonnoir dont la partie centrale est occupée par le passage de communication entre la cuve de filtration et l'enceinte de stockage, la cuve de filtration étant surmontée par un couvercle pourvu d'anses pour la suspension de l'ensemble, et la cuve de filtration comporte intérieurement une pluralité de toiles filtrantes superposées, de mailles de dimensions décroissantes de haut en bas.

On peut prévoir que chacune des toiles filtrantes sera supportée par une bague périphérique, dont le diamètre, décroissant du filtre supérieur vers les filtres inférieurs permet l'emboîtement à force des bagues contre la paroi tronconique dans des positions superposées, et les toiles filtrantes ont une forme générale conique dont le sommet est orienté vers le haut.

La toile filtrante supérieure peut recevoir une configuration conique dans sa partie centrale, le sommet étant orienté vers le haut, de façon concentrique aux toiles filtrantes inférieures, la partie conique centrale de la toile supérieure étant raccordée à une partie périphérique en forme de tronc de cône inversé et cette toile filtrante supérieure comporte des picots verticaux pour la rétention et l'immobilisation des caillots véhiculés éventuellement dans le sang provenant du sujet.

Selon une autre particularité le couvercle de la cuve de filtration comporte un soufflet pourvu à sa partie supérieure d'un bouchon amovible en matériau étanche mais souple tel qu'une résine élastomère permettant son remplissage en produits anticoagulants à l'aide d'une seringue et d'une aiguille traversant ledit bouchon, la manoeuvre du soufflet assurant la dispersion sous forme de brouillard desdits produits anti-coagulants dans la partie supérieure de l'enceinte de filtration.

Dans une variante de réalisation compactée la cuve de filtration est raccordée à la cuve inférieure contenant l'enceinte de collecte et de stockage par une ceinture sensiblement circulaire traversée selon deux passages diamètralement opposés par un canal tubulaire constituant le boisseau occupé par la clé de ladite vanne à plusieurs voies, la clé étant apte à être manipulée en rotation par une manette extérieure, ladite clé comportant deux canaux, dont un est apte à mettre communication en même temps l'enceinte de stockage avec l'enceinte de filtration par les passages colinéaires et appartenant respectivement à chacune des enceintes, l'autre canal étant apte à assurer en même temps la communication de l'enceinte de stockage avec la source de dépression (en assurant la mise en position d'aspiration) tandis que pour une seconde position de la manette et de la clé, les enceintes de filtration et de collecte sont isolées l'une de l'autre tandis que la chambre de collecte est mise en circulation avec une source de surpression constituée par exemple d'un soufflet manuel.

La poche constituant l'enceinte de collecte et de stockage peut contenir intérieurement une bourse remplie de produits anti-coagulants et dont la paroi est constituée d'une membrane en matériau élastiquement déformable et traversé par une multiplicité de pores, permettant aux produits anticoagulants situés à l'intérieur de ladite bourse de s'exsuder vers l'extérieur pour se mélanger au sang en cours de stockage, ladite bourse en matériaux élastiquement déformables étant appelée à prendre une position expansée (avec ouvertures des pores et passage des produits anti-coagulants) dans la phase de mise en dépression de l'enceinte de stockage.

La base de l'enceinte de stockage communique par un passage avec une chambre d'évacuation contenant des seconds moyens de filtration formés d'une toile filtrante en forme de cône la pointe étant orientée vers le bas et aboutissant à une sortie à laquelle sont raccordés des moyens de transfusion.

Outre les dispositions qui précèdent, l'invention comprend encore d'autres dispositions, qui ressortiront de la description qui va suivre, en se référant aux dessins annexés dans lesquels :

- la figure 1 est une vue éclatée du dispositif selon l'invention,
- la figure 2 est une vue en élévation verticale du dispositif conforme à l'invention, avec les parties composantes illustrées à la figure 1 assemblées entre elles,
- la figure 3 est une vue de dessus, avec arrachement partiel du dispositif représenté à la figure 2,
- la figure 4 est une vue en coupe verticale axiale du dispositif représenté à la figure 2,

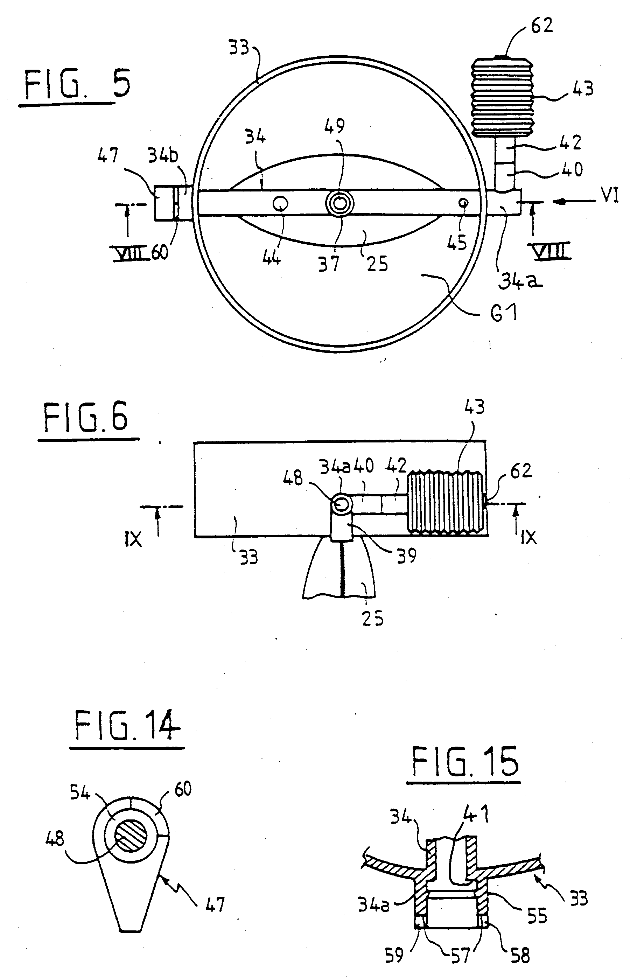

- la figure 5 est une vue de dessus de l'ensemble d'aspiration/refoulement du dispositif,
- la figure 6 est une vue en élévation, suivant VI et avec arrachement partiel de l'ensemble d'aspiration/refoulement illustrée à la figure 5,
- la figure 7 est une vue en coupe verticale axiale de l'ensemble d'aspiration/refoulement selon l'invention, telle que représentée à la figure 1, le boisseau de la vanne à plusieurs voies étant dépourvu de sa clé de commande conforme à l'invention.

- la figure 8 est une vue en coupe verticale axiale suivant le plan VIII de l'ensemble d'aspiration/refoulement illustrée à la figure 7, montrant la clé de la vanne en position d'aspiration du sang,
- la figure 9 est une vue en coupe horizontale suivant le plan IX de l'ensemble d'aspiration/refoulement illustrée à la figure 8, montrant le robinet de commande également en position d'aspiration,
- la figure 10 est une vue en élévation de la clé de la vanne à plusieurs voies représentée dans la position d'aspiration correspondant à la figure 9,
- la figure 11 est une vue en coupe verticale axiale de l'ensemble d'aspiration/refoulement dans laquelle la clé de la vanne est en position de mise en compression de la poche de collecte du sang, et donc de refoulement de ce dernier,
- la figure 12 est une vue en coupe horizontale de l'ensemble d'aspiration/refoulement précitée la clé étant dans la même position que celle représentée à la figure 13,
- la figure 13 est une vue en élévation de la clé représentée dans la position correspondant à la figure 11,
- la figure 14 est une vue frontale à plus grande échelle de la manette de la vanne à plusieurs voies,
- la figure 15 est une vue à plus grande échelle d'un détail de la portion du boisseau coopérant avec la manette du robinet de commande.

Le sang est prélevé et recueilli par aspiration au niveau de la plaie par une sonde ou dispositif connu (non représenté) et aboutissant par une tubulure souple raccordée par la section de tubulure 31 à une enceinte de filtration formée de la cuve 2. Cette cuve 2 est tronconique avec section décroissante vers le bas et génératrice (ou paroi latérale) 4 à faible pente. Le fond 5 de la cuve est en forme d'entonnoir, se terminant en son centre par un passage central 6 de raccordement à l'enceinte de collecte et de stockage formée de la poche souple 25 au sein de la cuve 18.

L'enceinte de filtration 2 comporte une série de filtres superposés et constitués des toiles 9,9a,9b,9c,9d dont la forme générale est un cône pointe en haut ; la toile supérieure 9 comportant une périphérie tronconique ouverte vers le haut (selon une coupe diamétrale en W). La disposition des filtres coniques 9a à 9c avec leurs sommets orientés vers le haut a pour but d'offrir aux matières filtrées une surface de dépôt plus grande à la périphérie des filtres.

La toile supérieure 9 est perforée uniformément sur toute sa surface (cf. les pores 12) qui est hérissée de picots verticaux 11. Ainsi, les gros débris sont retenus par la "forêt de picots" et le sang peut s'écouler entre les picots 11 et à travers les pores 12. La toile filtrante 9 est à mailles larges d'au moins 1 mm de diamètre, de façon que sur une surface de 2 mm carrés il y ait toujours au moins un orifice d'1 mm de diamètre et 3 à 4 picots qui l'entourent. Ces picots possèdent un relief irrégulier, avec aspérités, leur permettant d'accrocher plus facilement les "débris" qui passent à proximité. Leur irrégularité de relief est plus importante au sommet qu'à la base.

Au contraire, la taille de maille des toiles filtrantes 9a à 9c est plus fine par rapport à celle de la toile 9 et de plus en plus fine en passant de la toile 9a à la toile 9c. Les toiles sont montées chacune sur une bague périphérique et le diamètre moyen des bagues est décroissant dans un ordre tel qu'on puisse obtenir le positionnement des toiles filtrantes 9 et 9a à 9c par emboîtement à force à l'intérieur de la cuve conique 2 et contre la face interne de sa paroi 4 : de cette manière, chaque filtre ne peut occuper qu'une place bien définie, correspondant à l'ordre de filtrage prévu, et aucun risque d'erreur n'est possible dans le montage des filtres.

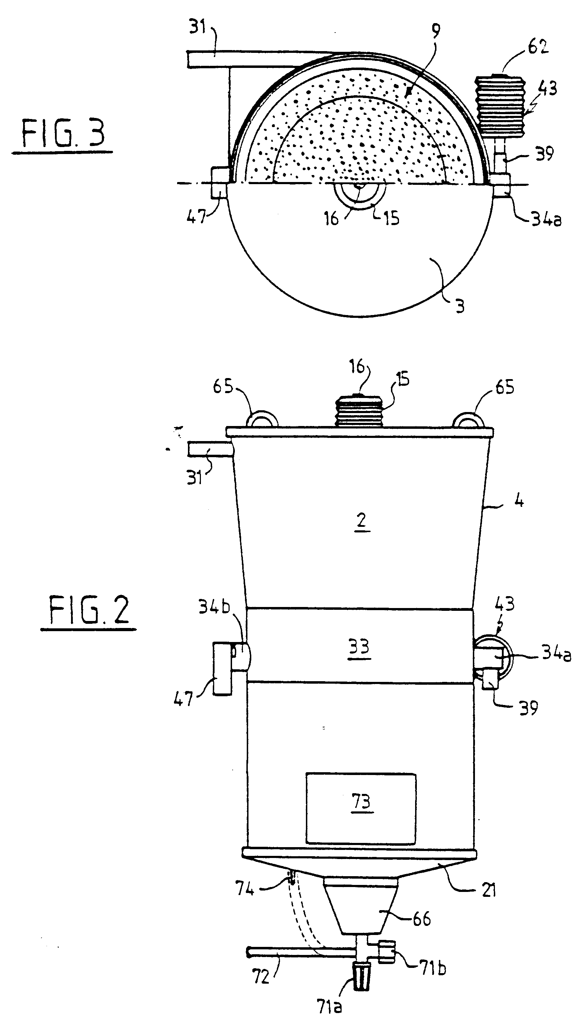

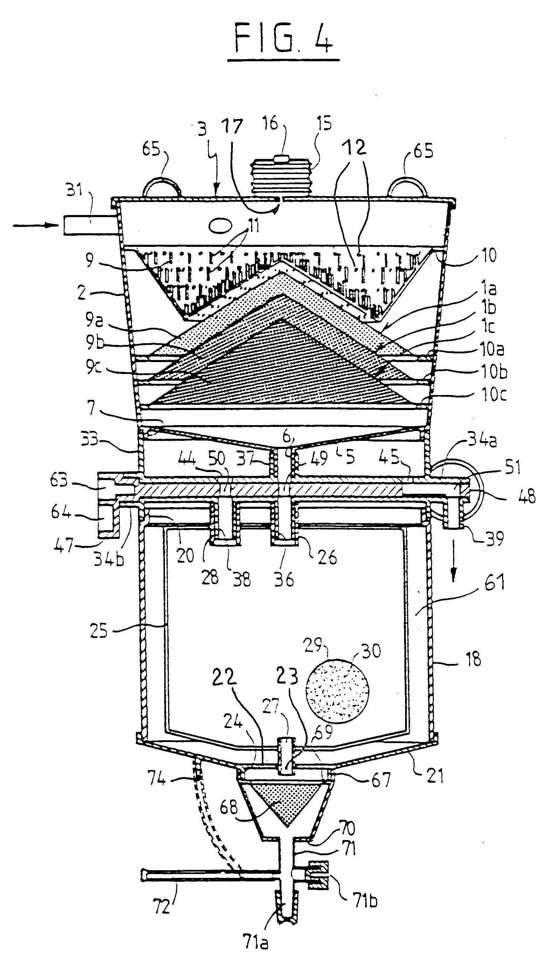

Le couvercle 3 permet de fermer la cuve 2 par emboîtement sur cette dernière. Il est fixé à cette cuve par collage. A la partie supérieure du couvercle 2 est disposé un soufflet 15 (cf. aussi les figures 2 et 4) destiné à être rempli d'un anti-coagulant à l'aide d'une seringue et de son aiguille (non représentées) par piquage au travers d'un bouchon en élastomère 16. Ce soufflet/réservoir permet la vaporisation (ou pulvérisation ou aérosolisation) dudit anti-coagulant avant et en cours d'aspiration du sang opératoire : à cet effet, le soufflet 15 de vaporisation des produits anti-coagulants communique avec un orifice 17 percé dans le couvercle 3 dans l'axe du soufflet.

Ce couvercle est muni avantageusement d'anses ou oeillets de suspension 65 permettant de maintenir le dispositif selon l'invention en position verticale pour assurer le refoulement par gravité du sang aspiré et collecté.

Il est avantageux d'équiper la cuve 2 d'une pochette en matière plastique (non représentée) fixée à la partie supérieure de cette cuve et contenant des flacons remplis de substances anti-coagulantes spécifiques ou de mélanges particuliers de ces dernières. A toutes fins utiles, cette pochette pourrait contenir également tout document spécifiant les mélanges et les dosages ainsi que les manoeuvres d'utilisation correcte du dispositif selon l'invention.

L'enceinte de collecte et de stockage formée de la poche 25 est située dans la cuve 18 fermée à sa base par un fond 21 sensiblement en forme d'entonnoir et qui présente une portion horizontale (ou disque) centrale 22 percée d'un orifice axial 23.

La cuve 18 contient une poche 25 en matière plastique souple, par exemple, en Triothyltriméthylate (TOTM), qui laisse passer l'oxygène et le CO et qui est fabriqué par la Société américaine CUTTER.

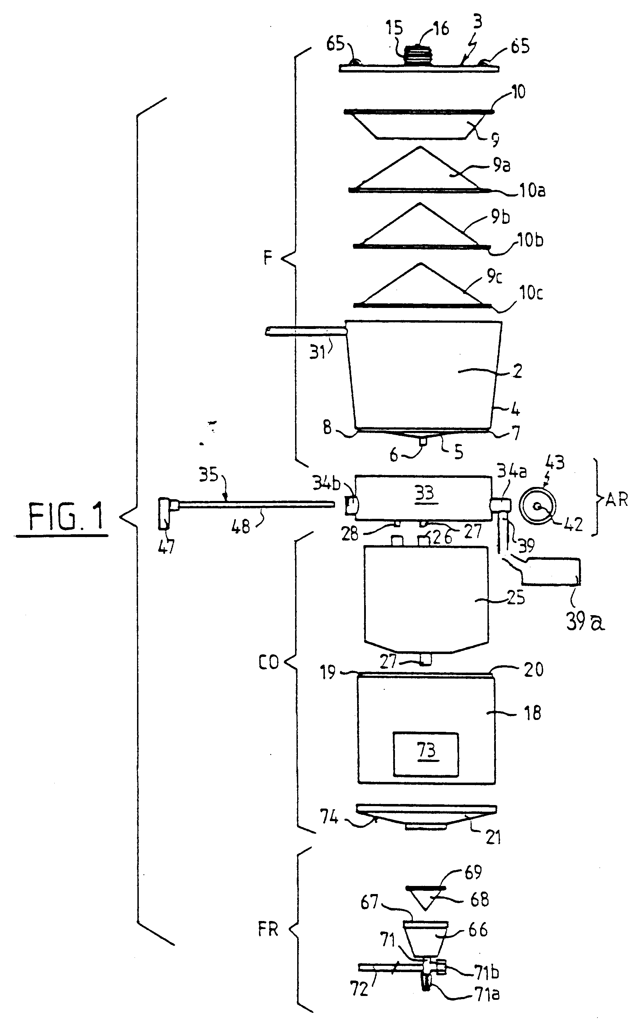

La poche 25 constituant l'enceinte de collecte du sang est équipée d'une pluralité d'embouts dénotés aux figures 1 et 4 par les références 26 à 28 et dont la fonction sera précisée plus loin. Cette poche 25 renferme une bourse 30 par exemple en forme de sphère dont la paroi est réalisée en matière plastique souple, cellulée et poreuse, ou bien en colloïdes poreux, et qui est remplie d'une substance anti-coagulante dont la libération a lieu dans la poche 25 comme décrit ci-après. Une ceinture sensiblement cylindrique 33 s'emboîte entre la cuve supérieure 2 et la cuve inférieure 18 et permet leur raccordement.

La fixation de cette ceinture 33 est obtenue par collage de manière à assurer la nécessaire étanchéité (il va de soi que, avant d'emboîter et coller la ceinture 33 par rapport aux cuves 2 et 18, ladite poche de collecte 25 est mise en place).

La ceinture 33 supporte en position diamètrale une pièce tubulaire 34 formant boisseau récepteur de la clé 48 constituant avec le boisseau une vanne à plusieurs voies.

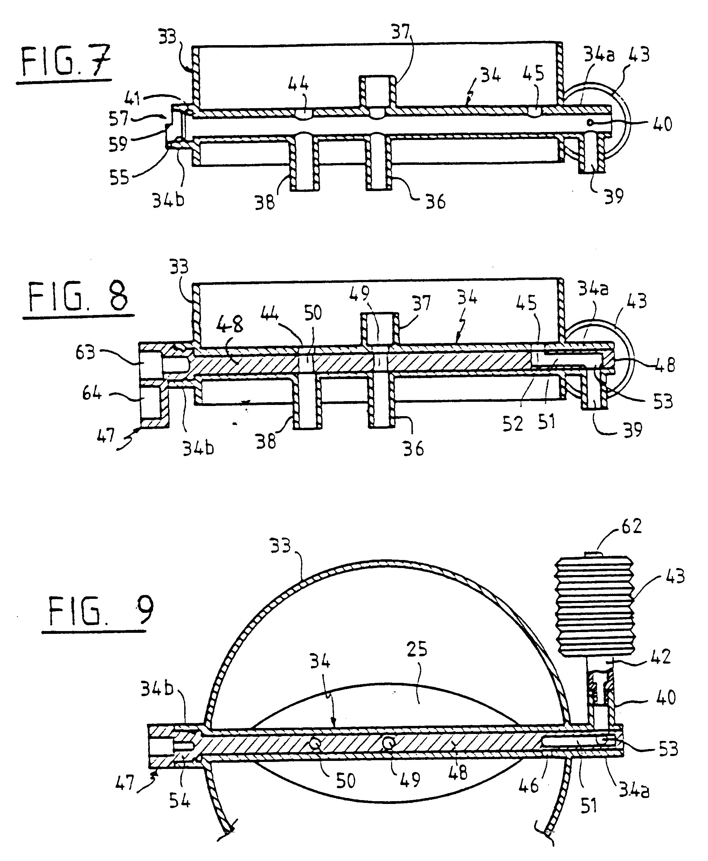

Le boisseau 34 se prolonge à l'extérieur de la ceinture 33 de part et d'autre par rapport à l'axe vertical du dispositif, de manière à définir une portion 34a de même diamètres intérieur et extérieur et une portion 34b dont les diamètres intérieur et extérieur sont plus grands que celui de la pièce tubulaire 34 de manière à définir un épaulement 41 (cf. les figures 7 et 15) faisant butée pour la clé 48.

Le boisseau comporte une pluralité de voies 36 à 40, parmi lesquels les voies 36 à 39 sont contenues dans un plan vertical passant par l'axe du boisseau 34 et sont perpendiculaires à cet axe, tandis que la voie 40 est contenue dans un plan horizontal passant également par l'axe dudit boisseau, tout en étant lui aussi perpendiculaire à cet axe.

Les voies 36 et 37 sont colinéaires et reçoivent par emboîtement les canaux également colinéaires 6 et 26 venus respectivement de l'enceinte de filtration 2 et de l'enceinte de collecte 25.

Une voie 38 du boisseau reçoit également par engagement le canal 28 venu de l'enceinte de collecte. Les voies 39 et 40 sont situées à l'extérieur de la ceinture 33 et sont portées par la portion extérieure 34a du boisseau 34. La voie 39, qui est dirigée vers le bas, est reliée à une source de vide 39a tandis que la voie 40 (qui est dirigée horizontalement et est à angle droit par rapport à la voie 39) reçoit la tubulure 42 d'un soufflet 43.

Dans la paroi du boisseau 34 sont ménagés aussi trois orifices 44 à 46, parmi lesquels l'orifice 44 est ménagé en position diamétralement opposée par rapport à la voie 38.

La clé 48 montée à rotation dans le boisseau 34, comporte une manette 47 et elle est réalisée en une matière plastique ayant des propriétés autolubrifiantes, tel que le polyéthylène à haute masse moléculaire ou autre, et engagée aves légère friction dans le boisseau 34; celui-ci présente comme la clé, une section cylindrique uniforme et constante d'un bout à l'autre pour assurer la parfaite étanchéité des circuits qui seront décrits ci-après. Dans la clé 48 sont ménagés des canaux transversaux 50 et 49 destinés à communiquer avec la voie 38 et l'orifice 44 et, respectivement, les voies diamétralement opposées 36, 37 du boisseau 34.

En outre, dans la clé 48 est ménagé un canal axial 51, borgne aux deux extrémités et ayant accès à la surface de la clé par deux orifices 52, 53 destinés à communiquer soit avec la voie 39 et l'orifice 45, soit avec le soufflet 43 et l'orifice 46 précités: donc, en position de montage le canal 51 comporte deux portions qui se situent de part et d'autre par rapport à la ceinture 33.

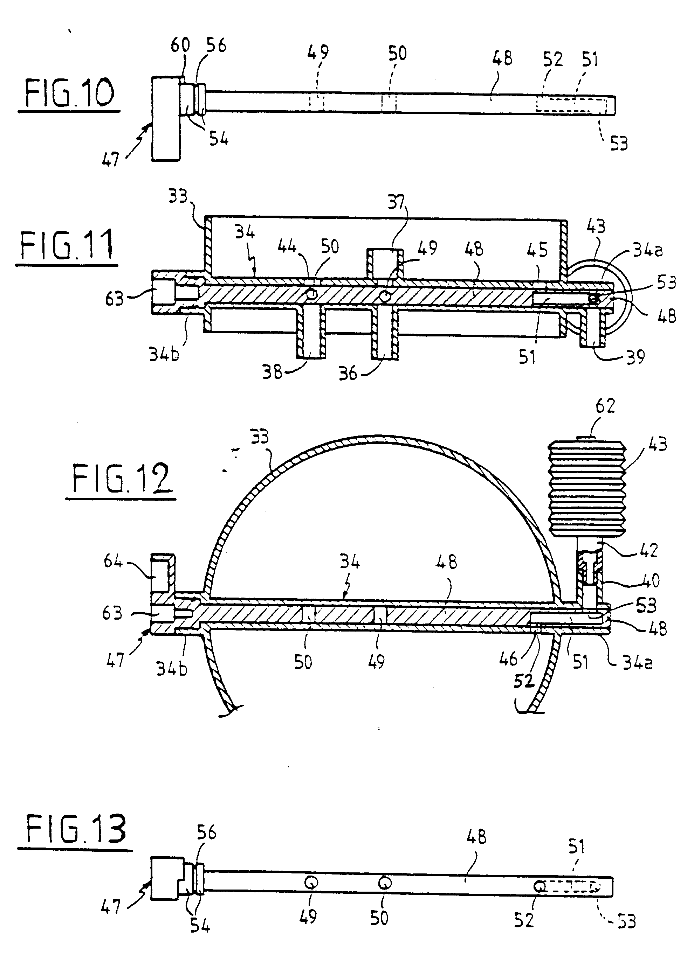

La manette de manoeuvre 47 de la clé 48, comporte des évidements d'allègement 63 et 64 et est perpendiculaire à la clé 48 à laquelle elle est raccordée par une portion cylindrique 54 destinée à s'emboîter dans la portion 34b du boisseau tubulaire 34 par encliquetage d'une nervure annulaire 55 (cf. les figures 9 et 15) de celle-ci dans une gorge annulaire 56 (cf. les figures 12 et 15) de la portion de raccord 54 susdite de la manette 47 de la clé 48.

A l'extrémité de la portion 34a est ménagée une gorge semicirculaire 57 (cf. les figures 9 et 15) définissant deux épaulements diamétralement opposés 58 et 59 (cf. la figure 15) de la portion 34b. La portion cylindrique 54 précitée comporte un ergot arqué 60, défini par un arc de 90°, qui est destiné à tourner dans la gorge semi-circulaire 57 et à venir en butée soit contre l'épaulement 58, soit contre l'épaulement 59 par rotation de la manette 47 d'un quart de tour.

Lorsque l'ergot 60 de la manette 47 est en butée contre l'épaulement 58, à savoir lorsque cette manette est dirigée verticalement vers le bas, les canaux 49 et 51 de la clef 48 sont en communication avec la voie 38 et l'orifice 44 et, respectivement, avec l'orifice 45 et la voie 39.

Les orifices 44 et 45 communiquent entre eux par l'intermédiaire de la chemise d'air 61 délimitée entre les surfaces internes de la cuve 18 et de la ceinture 33, le fond 5 de la cuve 2 ainsi que les surfaces externes de la poche 25. Il est clair que dans ces conditions, la clé 48 est en position d'aspiration, ce qui permet d'évacuer l'air contenu dans la poche 25 à travers la voie 38 formant reniflard et, par conséquent, de créer une dépression dans la poche, et donc dans la cuve 2, par l'intermédiaire du passage défini par le canal 50 de la clé et les voies 36 et 37 du boisseau 34.

Cette dépression se transmet aussi à la sonde ou canule d'aspiration par l'intermédiaire de la tubulure 31 de la cuve 2.

La voie 38 est avantageusement munie d'un filtre anti-bactérien de type classique (et non représenté), dont la porosité est de 0,10 à 0,25 microns.

La dépression réalisée dans la chemise 61 distend ladite membrane 29 de la bourse 30 remplie de substance anti-coagulante et contenue dans cette poche 25, élargissant ainsi les pores de la membrane et faisant céder sa structure, en sorte que le liquide anti-coagulant se libère et se mélange au sang. De toutes façons, la pulvérisation des substances anti-coagulantes contenues dans le soufflet 15 (cf. les figures 1 et 4) fait que celles-ci se répandent sur les parois internes du dispositif, notamment les filtres où elles se mélangent au sang.

Lorsque l'ergot 60 de la manette 47 est en butée contre l'épaulement 58, à savoir lorsque cette manette est horizontale, les canaux 49 et 50 de la clé ainsi que l'orifice 53 du canal borgne 51, ménagés dans la clé, sont obturés par la paroi du boisseau 34, tandis que l'orifice 52 du canal borgne 51 est en communication avec l'orifice 46 du boisseau 34. Dans ces conditions, le soufflet 43 est en communication avec ladite chemise 61 dans laquelle peut être donc soufflé de l'air pour créer une surpression dans cette chemise et donc comprimer la poche 25 remplie de sang de manière à permettre la transfusion vers le corps du sujet. Le soufflet 43 est amovible et se verrouille facilement dans l'embout 40 susdit de la pièce 34 grâce à la présence de rainures de blocage ménagées sur 1/4 de tour (non représentées parce que ce système de verrouillage est connu des techniciens en la matière), dans sa tubulure de raccordement 42, l'étanchéité étant obtenue à l'aide d'un joint souple.

Une soupape 62 d'admission d'air permet l'entrée d'air lors de l'actionnement du soufflet 43. Le cas échéant, on peut dévisser ce soufflet pour faire baisser la pression dans la chemise d'air 61 lorsque le débit est relativement élevé. La position dans laquelle se trouve la clé dans ces conditions est une position assurant donc le refoulement amélioré du sang aspiré et collecté dans la poche 25 vers le malade.

La base de la cuve 18 comporte une cuvette de filtration 66 tronconique qui se prolonge à la base supérieure, de plus grand diamètre, dans une couronne 67 sensiblement cylindrique destinée à s'emboîter autour de la nervure annulaire 24 faisant saillie de la portion inférieure, centrale et horizontale, du fond 21 de la cuve 18 contenant la poche ou enceinte de collecte de sang 25. L'embout inférieur 27 de cette dernière est enfilé dans l'orifice central 23 ménagé dans le fond 21 précité. L'étanchéité nécessaire entre la cuve 18 et la cuvette 66 ainsi qu'entre l'embout 27 et le fond 21 est obtenue par collage.

A l'intérieur de la cuvette 66 est logé un filtre "chaussette" composé d'un textile en matière plastique 68 en forme de cône dont les mailles présentent un diamètre de 75 (ou 100,150,200) $\mu$m et dont le sommet est orienté vers le bas, dans le sens de la cuvette tronconique 66, et qui est fixé, par collage ou soudage, à une bague 69 mince et cylindrique dont le diamètre extérieur est sensiblement égal au diamètre intérieur de la couronne 67 : ainsi le filtre se positionne de lui-même à la partie haute de la cuvette 66. Cette cuvette 66 est aussi un moyen permettant outre la filtration du sang avant son passage dans la ligne transfusionnelle, une première libération des bulles d'air qui pourraient être transportées avec le sang, étant entendu que la chambre du transfuseur branché sur l'appareil, jouera aussi ce rôle dans un deuxième temps, et qu'un piège à bulles peut être ensuite placé (précaution supplémentaire) sur la ligne classique de transfusion.

Une tubulure 71, à deux voies ou embouts 71a et 71b, fait saillie vers le bas à partir de la base 70 de la cuvette 66. L'embout 71a permet le raccordement du dispositif selon l'invention à un transfuseur classique, à savoir à une tubulure classique de transfusion connue des techniciens en la matière (et non représentée). L'embout 71b, qui est du type connu sous le nom de marque de fabrique "Luer-lock", permet le raccordement à une pompe à galets (celle-ci non plus représentée, car elle est connue des techniciens en la matière) destinée à mobiliser le sang transféré vers le malade.

La tubulure 71 est également pourvue d'un capillaire 72 rendu borgne à son extrémité libre, par exemple par sertissage, et destiné à permettre le prélèvement de sang de la poche de collecte 25 nécessaire pour la vérification du groupe sanguin du malade; ceci se pratique en sectionnant le bout serti du capillaire 72 et en appliquant quelques gouttes de sang sur des tests qui peuvent être collés sur une étiquette d'identification, telle que celle schématiquement illustrée aux figures 1 et 2 et dénotée par la référence 73. Ces tests peuvent avantageusement être protégés par une fine pellicule en matière plastique qui en garantit un usage unique.

Une fois la vérification du groupe sanguin effectuée, le capillaire 72 peut être refermé en l'engageant sur un picot 74 qui fait saillie vers le bas, à partir du fond 21 de la cuve 18, et qui sert de bouchon. Le diamètre de ce capillaire-tube plastique permet d'y introduire l'embout normalisé d'une seringue stérile afin de prélever par ce moyen, outre le sang nécessaire pour le test de groupage, des quantités de sang plus importantes permettant, hémocultures, vérification des facteurs de la coagulation, coagulabilité, etc....

Le dispositif d'auto-transfusion selon l'invention est entièrement stérile intérieurement et extérieurement et peut être avantageusement livré dans un emballage hermétique. Réalisé en matière plastique bio-compatibles, il ne sert qu'à une seule collection (usage unique) d'une quantité de sang ne dépassant pas 500 à 800 cm$^3$.

Il peut être aisément détruit par incinération après utilisation.

Il permet aussi de surveiller facilement tous les évènements qui ont lieu à l'intérieur du dispositif, par observation à travers ses parois : à cet effet, il est entièrement translucide et est réalisé à partir de matériaux tels que le polyéthylène, le PVC rigide et le polycarbonate et généralement en tout matériau hémocompatible.

De préférence, et à titre indicatif, son encombrement ne dépasse pas 48 cm de longueur et 24 cm de largeur (ces dimensions étant entendues comme dimensions hors-tout).

Ci-après est résumé le fonctionnement du dispositif selon l'invention, qui est basé sur le recueil du sang hémorragique en zone opératoire : il procède par aspiration de ce sang, puis extraction, sur des filtres successifs, de tous débris et agrégats, permettant ainsi de ne conserver que les produits originaux du sang ; enfin, le sang, stocké dans une poche-réceptacle, est encore filtré lors de son départ en sortie du dispositif, avant de passer dans la tubulure de transfusion, qui permet de le réinjecter au malade.

De façon plus précise, dans la position de fonctionnement (cf. la figure 2), le dispositif qui est stérile à l'origine est suspendu verticalement par les anses 65. L'embout 31 est relié à la sonde d'aspiration aux mains du chirurgien. L'embout 39 est relié à une source de vide du type disponible dans chaque hôpital ou à un dispositif permettant d'obtenir une pression négative (ce peut être une pompe électrique à galets d'un modèle transportable et connu en soi, en cas d'utilisation du set hors de toute installation technique). La manette 47 est en position verticale, dirigée vers le bas, donc le reniflard 38 et la voie 36 sont ouverts permettant ainsi le passage du sang depuis la cuve de filtrage 2 vers la poche ou enceinte de collecte 25. Le canal 51 est en communication avec l'embout 39 relié à la source de vide.

Plusieurs pressions sur le soufflet 15 font vaporiser en aérosolisation de très petites particules, dont le diamètre est compris entre 5 et 10 $\mu$m de liquide anti-coagulant sur les filtres contenus dans la cuve de filtrage 2, et par écoulement, dans la poche 25. Chaque fois que le chirurgien aspire le sang, des débris peuvent être aussi aspirés. L'arrivée du sang avec une certaine vitesse, se fait sur les parois supérieures de la cuve de filtrage susdite. Les particules les plus lourdes et les plus solides perdent leur vitesse avant les liquides et se déposent au fond de la première toile filtrante 9 à mailles larges. Pour éviter un encrassement trop rapide de celle-ci, un ensemble de picots 11 serrés retient les éléments les plus épais. Les trois autres toiles filtrantes 9a à 9c, à mailles de plus en plus serrées, complètent le filtrage du sang. Le sang filtré arrive au fond de la cuve 2 et passe par l'embout 6 dans la poche 25. L'aspiration provoquée par la voie 39 crée une dépression dans la chemise 61 maus aussi dans la poche 25.

La dépression ouvrant par remplissage de sang la poche 25, tend à distendre les parois de la baudruche 29 et, par conséquent, à élargir le diamètre des pores et à faire céder sa structure cellulée; le liquide auto-coagulant se libère et se mélange au sang.

Avant de transfuser ce sang (si l'on se trouve à distance chronologique du recueil), des tests de compatibilité isogroupe peuvent être réalisés. Ils sont prévus sur l'étiquette d'identification 73 protégée par un film plastique à arracher lors de l'utilisation. Le prélèvement pour le test se fait en sectionnant le capillaire 72 dont la longueur permet d'arriver au test. Le prélèvement effectué, le capillaire est fermé par le picot 74.

La transfusion est normalement réalisée immédiatement :
- par l'intermédiaire d'une tubulure de transfusion classique (munie d'une chambre avec filtre) dont le perforateur est introduit dans l'embout 71a,
- ou bien, par l'intermédiaire d'une tubulure qui est fixée sur le "Luer-Lock" femelle 71b et qui est ensuite introduite dans une pompe à galets classique, provoquant l'aspiration du sang dans la tubulure, elle-même connectée à un catheter veineux au niveau du malade.

En alternative, le stockage au froid du sang recueilli dans la poche 25 permet une utilisation ultérieure de ces mêmes réserves de sang.

Dans ce qui suit, on décrit deux situations extrêmes, données essentiellement à titre d'exemples illustratifs et non limitatifs, dans lesquelles le dispositif d'auto-transfusion selon l'invention est susceptible d'être utilisé, chaque exemple montrant le fonctionnement de ce dispositif.

Exemple d'utilisation n° 1 :

Cette première situation extrême correspond au cas d'un malade appartenant au groupe sanguin AB rhésus négatif, qui présente les signes évidents d'une rupture hémorragique d'anévrysme artériel et qui est transporté d'urgence dans le Service de Chirurgie Générale d'un hôpital, dont le Centre de Transfusion Sanguine ne dispose plus, malgré les efforts et les recherches, de sang compatible pour transfusion dans un bref délai. Toutefois, étant donné que la perte de sang fait effondrer la tension artérielle du malade, il est clair que, dans ce cas, il faut intervenir rapidement pour arrêter l'hémorragie, et ce sans avoir la possibilité de transfuser le malade avec du sang compatible : il s'agit donc bien d'une situation, extrême certes, dans laquelle l'utilisation du dispositif conforme à l'invention peut rendre un service essentiel.

A cet effet, le malade est installé en salle d'opération, où les champs opératoires stériles et le matériel stérile d'intervention sont mis en place, et une infirmière branche l'aspiration générale de la salle d'opération sur l'embout prévu à cet effet sur le dispositif d'auto-transfusion qui est, donc, prêt à fonctionner pour aspirer le sang perdu par le malade lors de l'intervention. Pour ce faire, le chirurgien dégage (après ouverture de la paroi abdominale), au moyen d'une canule d'aspiration stérile, la zone hémorragique où l'anévrysme fuit à chaque systole, laissant échapper un filet de sang.

Tout le sang que le chirurgien aspire à l'aide de la canule passe dans un tuyau stérile plastique souple (PVC), de préférence transparent, qui fait suite à la canule et dont le diamètre interne peut varier entre 5 et 10 mm.

Le sang aboutit dans la cuve de filtrage du dispositif grâce à la source d'aspiration qui est branchée sur ce bac et qui y crée le vide. En ce qui concerne les autres opérations, celles-ci peuvent être déduites de la description qui précède.

Exemple d'utilisation n° 2

Cette deuxième situation extrême se réfère au cas où un tremblement de terre a eu lieu avec destruction d'immeubles, y compris les hôpitaux, et des milliers de blessés souffrant de traumatismes divers, dont ceux de l'écrasement, tels qu'écrasements thoraciques, éclatements de la rate, contusions hémorragiques du bassin et de l'abdomen, traumatismes hémorragiques du rein, écrasements artériels et ruptures des artères des membres.

Dans la plupart de ces cas, le pronostic est désespéré à cause de l'absence de grandes quantités de sang pour transfusions, surtout lorsque les hôpitaux et C.T.S. sont eux-mêmes détruits, ce qui permet d'apprécier les avantages liés à l'emploi du dispositif d'auto-transfusion conforme à la présente invention, qui permet de restituer aux blessés leur sang hémorragique, c'est-à-dire de pallier à l'impossibilité matérielle de transfusions, au fur et à mesure que l'intervention chirurgicale qui rentre nécessairement dans le cadre d'une chirurgie sanglante d'urgence a lieu dans les Centres d'Interventions Chirurgicales les plus proches des endroits où la catastrophe s'est produite.

On doit noter que certaines plaies fréquentes en Traumatologie, au premier abord peu graves pour la vie des sujets, comme de larges plaies du scalp ou cuir chevelu, peuvent être à l'origine de pertes sanglantes irréductibles et prolongées, conduisant ces sujets vers une mort par hémorragie si l'on ne compense pas ces pertes de sang immédiatement et aussi longtemps qu'elles persistent.

En ce qui concerne la description du fonctionnement du dispositif, il est évident qu'elle est la même que celle indiquée dans la situation décrite dans l'exemple 1 ; toutefois, dans ce cas, le dispositif peut être, si l'on ne dispose pas d'une source d'aspiration comme celle figurant dans tous les blocs opératoires, relié à une pompe électrique à galets ou toute autre pompe d'aspiration de bloc sanitaire mobile de campagne et d'urgence.

Bien que la description des exemples d'utilisation ait été limitée aux cas n° 1 et n° 2 susdits, il va de soi qu'il existe de nombreuses situations intermédiaires ou équivalentes aux situations décrites dans les exemples précédents dans lesquelles le dispositif selon l'invention est aussi avantageusement susceptible d'être utilisé.

Le dispositif selon l'invention ne présente pas de désavantages pour les malades, si l'on considère que :

- les produits anti-coagulants qui y sont utilisés sont d'usage courant depuis des décennies et qu'en particulier l'Héparine, qui est utilisée dans le dispositif et passe dans la circulation du malade lors de la transfusion, peut être rapidement et efficacement neutralisée quant à ses effets anti-coagulants systémiques, grâce à l'utilisation de Protamine injectable ;
- toutes les matières plastiques utilisées relèvent de la meilleure biocompatibilité et, qu'en dehors du stockage au froid (pour lequel la poche présente dans le dispositif est une utilisation habituelle), le temps de contact du sang avec ces matériaux est bref ;
- les filtres équipant le dispositif d'auto-transfusion garantissent contre le passage d'agrégats ou de particules étrangères ou constituées, lors de la transfusion ultérieure du sang récolté, et
- le filtre anti-bactérien du reniflard est une barrière contre la contamination bactérienne de l'air, et que l'on sait pertinemment que malgré l'ensemble des précautions prises en salle opératoire, la contamination est toujours présente, justifiant chez tous ces malades des traitements antibiotiques préventifs et appropriés selon la nature des germes susceptibles d'avoir contaminé les malades. Le dispositif est ici d'autant plus intéressant qu'il permet au malade de recouvrer une partie de ses défenses naturelles présentes dans le sang circulant et qui seraient sinon perdues pour lui.

L'invention permet de réaliser un ensemble d'autotransfusion compacté et de faible encombrement: réalisé en matière de synthèse hémocompatible et même en matériau à propriétés anticoagulantes (contenant des hépraines); il est d'un prix de revient modéré permettant un usage unique et une grande sécurité d'emploi.

Sans faire cependant appel à des systèmes complexes, encombrant, coûteux et d'utilisation délicate, le dispositif selon l'invention permet un fonctionnement quasi automatique et ne nécessite pas une infrastructure lourde. Il assure une parfaite sécurité face à ses deux étapes de filtration dont une première étape amont est particulièrement efficace avec la mise en jeu de lits de filtration superposés. De plus la mise en oeuvre de moyens anticoagulants se fait de façon régulière et constante et ceci dès la phase de recueil et filtration, cette introduction de moyens anticoagulants étant poursuivie de façon parfaitement dosée lors de la phase de collecte et de stockage.

L'appareil ainsi réalisé peut être stocké sous forme d'ensemble compact dans un emballage stérile immédiatement prêt à l'emploi et susceptible d'être réduit après usage.

**Revendications**

1. Dispositif en vue de la récupération et de la réinjection dans l'organisme du sujet de son propre sang en vue de compenser une hémorragie, comportant:

a) des moyens de récupération du sang par aspiration aptes à être positionnés à proximité immédiate du point de rupture des vaisseaux

b) une première enceinte de filtration (2) apte à recueillir le sang provenant des moyens de récupération, ladite enceinte (2) étant pourvue de lits filtrants (9, 9a, 9b, 9c)

c) une seconde enceinte (25) de collecte et de stockage située en dessous de l'enceinte de filtration (2), l'enceinte (25) de collecte et de stockage étant apte à recueillir le sang s'écoulant par gravité et dépression depuis l'enceinte de filtration (2), la base de l'enceinte (25) de collecte et de stockage communiquant par un tube de sortie inférieur (71a) avec des moyens de rétrotransfusion dans le système circulatoire du sujet, et dans lequel,

d) ladite enceinte de collecte et de stockage communique par son sommet d'une part avec une source de dépression (39a) et d'autre part avec l'enceinte de filtration (2), la dépression susceptible d'être engendrée au sein de l'enceinte de stockage se communiquant à l'enceinte de filtration et permettant l'aspiration et la filtration sous vide partiel du sang et son écoulement depuis l'enceinte de filtration (2) vers l'enceinte de collecte et de stockage (25)

e) ladite enceinte de collecte et de stockage (25) communique également par son sommet avec une source de surpression (43) apte à provoquer par surpression dans la phase gazeuse de ladite enceinte (25) la mise en circulation du sang vers des moyens de réinjection par transfusion dans le système sanguin du sujet caractérisé en ce que

f) ladite enceinte de filtration (2) comporte à son sommet une source (15), communiquant avec ladite enceinte de filtration (2), de produits anti-coagulants permettant la dispersion desdits produits anti-coagulants sous forme de brouillard aptes à remplir la phase gazeuse de ladite enceinte de filtration au-dessus et en amont des moyens de filtration prévus dans ladite enceinte (2)

g) l'enceinte (25) de collecte et de stockage comporte en son sein une seconde réserve (30) d'agents anti-coagulants, ladite réserve étant immergée dans la réserve de sang en cours de stockage, ladite réserve comportant des moyens de dispersion permettant la diffusion desdits agents dans le sang en cours de stockage

h) la communication entre les deux enceintes respectivement de filtration (2) et de collecte et stockage (25) d'une part entre elles et d'autre part respectivement avec les sources de dépression (39a) et de surpression (43) étant commandée par une vanne unique à plusieurs voies (34, 48) ladite vanne étant apte pour une première position à isoler l'enceinte de stockage (25) de la source de surpression en mettant cette enceinte en communication avec la source de dépression et avec l'enceinte de filtration, position pour laquelle le sang est ainsi aspiré vers l'enceinte de filtration et au-delà vers l'enceinte de collecte, la vanne (34, 48) étant apte pour la seconde position à isoler l'enceinte de collecte et stockage (25) par rapport à la source de dépression (39a) et par rapport à l'enceinte de filtration (42), en mettant ladite enceinte de collecte et stockage (25) en communication avec la source de surpression (43) position pour laquelle la surpression engendrée au niveau de l'enceinte de collecte et stockage provoque le déplacement du sang vers les moyens de rétro-transfusion.

2. Dispositif selon la revendication 1, caractérisé en ce que l'enceinte de collecte et de stockage (25) est constituée d'une poche en matière plastique souple apte à recevoir le sang provenant de l'enceinte de filtration, cette poche étant reliée par ladite vanne (33, 34) à sa partie supérieure avec l'enceinte de filtration (2) et communiquant par sa base avec un circuit d'évacuation comportant notamment des seconds moyens de filtration (68), ladite enceinte de stockage (25) étant positionnée à l'intérieur d'une cuve rigide (18, l'espace entre la paroi de la cuve et ladite enceinte ou poche (25) formant une chemise d'air (61) ladite chemise d'air étant en communication par un passage formant reniflard avec ladite poche (25) et étant mise en communication par son sommet et par l'intermédiaire de la vanne à plusieurs voies (33, 34) alternativement avec la source de dépression ou la source de surpression suivant le positionnement de ladite vanne.

3. Dispositif selon l'une quelconque des revendications 1 ou 2, caractérisé en ce que la cuve de filtration (2) a une forme générale tronconique, la petite base étant en position inférieure et comporte un fond en forme d'entonnoir (7) dont la partie centrale (6) est occupée par le passage de communication entre la cuve de filtration (2) et l'enceinte de collecte (25), la cuve de filtration étant surmontée par un couvercle (3) pourvu d'anses (65) pour la suspension de l'ensemble, et la cuve de filtration comporte intérieurement une pluralité de toiles filtrantes superposées (9, 9a, 9b, 9c), de mailles de dimensions décroissantes de haut en bas.

4. Dispositif selon la revendication 3, caractérisé en ce que chacune des toiles filtrantes est supportée par une bague périphérique (10, 10a, 10b, 10c), dont le diamètre, décroissant du filtre supérieur vers les filtres inférieurs permet l'emboîtement à force des bagues contre la paroi tronconique dans des position superposées.

5. Dispositif selon l'une quelconque des revendications 1 à 4, caractérisé en ce que les toiles filtrantes ont une forme générale conique dont le sommet est orienté vers le haut.

6. Dispositif selon l'une quelconque des revendications 3 à 5, caractérisé en ce que la toile filtrante supérieure(9) a une configuration conique dans sa partie centrale, le sommet étant orienté vers le haut, de façon concentrique aux toiles filtrantes inférieurs (9a, 9b, 9c), la partie conique centrale de la toile supérieure (9) étant raccordée à une partie périphérique en forme de tronc de cône inversé et cette toile filtrante supérieure comporte des picots verticaux (11) pour la rétention et l'immobilisation des débris organiques et caillots véhiculés éventuellement dans le sang provenant du sujet.

7. Dispositif selon l'une quelconque des revendications 1 à 6, caractérisé en ce que le couvercle (3) de la cuve de filtration (2) comporte un soufflet (15) pourvu à sa partie supérieure d'un bouchon amovible (16) en matériau étanche mais souple tel qu'une résine élastomère permettant son remplissage en produits anti-coagulants à l'aide d'une seringue et d'une aiguille traversant ledit bouchon (16), la manoeuvre du soufflet assurant la dispersion sous forme de brouillard desdits produits anti-coagulants dans la partie supérieure de l'enceinte de filtration (2).

8. Dispositif selon l'une quelconque des revendications 1 à 7, caractérisé en ce que la cuve de filtration (2) est raccordée à la cuve inférieure (18) contenant l'enceinte de collecte et de stockage par une ceinture sensiblement circulaire (33) traversée selon deux passages diamétralement opposés par un canal tubulaire constituant le boisseau (34) occupé par la clé (48) de ladite vanne à plusieurs voies, la clé étant apte à être manipulée en rotation par la manette extérieure (47), ladite clé comportant deux canaux (49) et (50) aptes à mettre en communication en même temps l'enceinte de filtration (2) par les passages colinéaires respectivement (6) et (26) appartenant à chacune des enceintes, le passage (50) étant apte à assurer en même temps la communication de l'enceinte de collecte (25) avec la source de dépression (39a) (en assurant la mise en position d'aspiration) tandis que pour une seconde position de la manette (47) et de la clé (48), les enceintes de filtration et de collecte sont isolées l'une de l'autre.

9. Dispositif selon l'une quelconque des revendications 1 à 8, caractérisé en ce que la poche (25) constituant l'enceinte de collecte et de stockage contient intérieurement une bourse (30) remplie de produits anti-coagulants et dont la paroi (29) est constituée d'une membrane en matériau élastiquement déformable et traversé par une multiplicité de pores, permettant aux produits anti-coagulants situés à l'intérieur de ladite bourse de s'exsuder vers l'extérieur pour se mélanger au sang en cours de stockage, ladite bourse en matériau élastiquement déformable étant appelée à prendre une position expansée (avec ouverture des pores et passage de produits anti-coagulants) dans la phase de mise en dépression de l'enceinte de stockage.

10. Dispositif selon l'une quelconque des revendications 1 à 9, caractérisé en ce que la base de l'enceinte de collecte (25) communique par un passage (27) avec une chambre d'évacuation (70) contenant des seconds moyens de filtration formés d'une toile filtrante en forme de cône la pointe étant orientée vers le bas et aboutissant à une sortie (71a) à laquelle sont raccordés des moyens de transfusion.

11. Dispositif selon l'une quelconque des revendications 1 à 10, caractérisé en ce qu'il comporte une cuve (2) constituant l'enceinte de filtration en position supérieure et solidaire d'une seconde cuve (18) contenant intérieurement l'enceinte de collecte (25), les deux cuves superposées étant conformées sous forme de corps de révolution coaxiaux et dont les parois se prolongent sensiblement étant solidaires l'une de l'autre.

12. Dispositif selon la revendication 11, caractérisé en ce que la cuve supérieure (2) et la cuve inférieure (18) sont réalisées en matière synthétique transparente et hémocompatible.

## Claims

1. Device for recuperating and reinjecting into the organism of the subject his/her own blood, with a view to compensating a haemorrhage, comprising:
   a) means for recuperating the blood by suction, adapted to be positioned in the immediate proximity of the point of rupture of the vessels,
   b) a first filtration vessel 2 adapted to collect the blood coming from the recuperation means, said vessel 2 being provided with filtering beds 9, 9a, 9b, 9c,
   c) a second vessel 25 for collection and storage, located beneath the filtration vessel 2, the collecting and storing vessel 25 being adapted to collect the blood flowing by gravity and depression from the filtration vessel 2, the base of the collecting and storage vessel 25 communicating by a lower outlet tube 71a with means for retro-transfusion into the patient's circulatory system,
and in which
d) said collecting and storage vessel communicates by its apex, on the one hand, with a source of depression 39a and, on the other hand, with the filtration vessel 2, the depression capable of being generated within the storage vessel being communicated to the filtration vessel and allowing suction and filtration under partial vacuum of the blood and flow thereof from the filtration vessel 2 towards the collection and storage vessel 25,
e) said collection and storage vessel 25 also communicates by its apex with a source of excess pressure 43 adapted to provoke by excess pressure in the gaseous phase of said vessel 25, the circulation of the blood towards means for reinjection by transfusion into the patient's blood system,
characterized in that
f) said filtration vessel 2 comprises at its apex a source 15, communicating with said filtration vessel 2, of anti-coagulant products allowing the dispersion of said anti-coagulant products in the form of mist adapted to fill the gaseous phase of said filtration vessel above and upstream of the filtration means provided in said vessel 2
g) the collection and storage vessel 25 comprises at its centre a second reserve 30 of anti-coagulant agents, said reserve being immersed in the reserve of blood being stored, said reserve comprising dispersion means allowing diffusion of said agents in the blood being stored
h) communication between the two vessels for filtration (2) and collection and storage (25) respectively, on the one hand, together and, on the other hand, respectively with the sources of depression 39a and of excess pressure 43 being controlled by a single multi-way valve 34, 48, said valve being adapted, for a first position, to isolate the storage vessel 25 from the source of excess pressure, by placing this vessel in communication with the source of depression and with the filtration vessel, position for which the blood is thus sucked towards the filtration vessel and beyond towards the collecting vessel, the valve 34, 48 being adapted, for the second position, to isolate the collection and storage vessel 25 with respect to the source of depression 39a and with respect to the filtration enclosure 42, by placing said collection and storage vessel 25 in communication with the source of excess pressure 43, position for which the excess pressure generated at the level of the collection and storage vessel provokes displacement of the blood towards the retrotransfusion means.

2. Device according to Claim 1, characterized in that the collection and storage vessel 25 is constituted by a bag of supple plastics material adapted to receive the blood coming from the filtration vessel, this bag being connected by said valve 33, 34, at its upper part, with the filtration vessel 2 and communicating by its base with an evacuation circuit comprising in particular second filtration means 68, said storage vessel 25 being positioned inside a rigid tank 18, the space between the wall of the tank and said vessel or bag 25 forming an air jacket 61, said air jacket being in communication by a passage forming breather with said bag 25 and being placed in communication by its apex and via the multiway valve 33, 34 alternately with the source of depression or the source of excess pressure depending on the positioning of said valve.

3. Device according to either one of Claims 1 or 2, characterized in that the filtration vessel 2 has a generally truncated form, the small base being in lower position and comprises a bottom in the form of a funnel 7 of which the central part 6 is occupied by the communication passage between the filtration vessel 2 and the collection vessel 25, the filtration vessel being surmounted by a cover 3 provided with handles 65 for suspension of the assembly, and the filtration vessel internally comprises a plurality of superposed filtering cloths 9, 9a, 9b, 9c with meshes of downwardly decreasing dimensions.

4. Device according to Claim 3, characterized in that each of the filtering cloths is supported by a peripheral ring 10, 10a, 10b, 10c, whose diameter, decreasing from the upper filter towards the lower filters, allows forcefit of the rings against the truncated wall in superposed positions.

5. Device according to one of Claims 1 to 4, characterized in that the filtering cloths are generally conical in form, with upwardly oriented apex.

6. Device according to any one of Claims 3 to 5, characterized in that the upper filtering cloth 9 has a conical configuration in its central part, the apex being oriented upwardly, concentrically with respect to the lower filtering cloths 9a, 9b, 9c, the central conical part of the upper cloth 9 being connected to a peripheral part in the form of an inverted conical frustum and this upper filtering cloth comprises vertical barbs 11 for retaining and immobilizing organic débris and clots possibly conveyed in the blood coming from the patient.

7. Device according to any one of Claims 1 to 6, characterized in that the cover 3 of the filtration vessel 2 comprises a bellows 15 provided in its upper part with a removable plug 16 made of tight but supple material such as an elastomer resin allowing it to be filled with anti-coagulant products with the aid of a syringe and needle passing through said plug 16, manoeuvring of the bellows ensuring dispersion in the form of mist of said anti-coagulant products in the upper part of the filtration vessel 2.

8. Device according to any one of Claims 1 to 7, characterized in that the filtration vessel 2 is connected to the lower tank 18 containing the collection and storage vessel by a substantially circular belt 33 traversed along two diametrally opposite passages by a tubular channel constituting the casing 34 occupied by the key 48 of said multi-way valve, the key being adapted to be manipulated in rotation by the outer handle 47, said key comprising two channels 49 and 50 adapted to place in communication at the same time the filtration vessel 2 by the colinear passages respectively 6 and 26 belonging to each of the vessels, the passage 50 being adapted to ensure at the same time communication of the collection vessel 25 with the source of depression 39a (ensuring placing into position of suction), whilst, for a second position of the handle 47 and of the key 48, the filtration and collection vessels are isolated from each other.

9. Device according to any one of Claims 1 to 8, characterized in that the bag 25 constituting the collection and storage vessel internally contains a pouch 30 filled with anti-coagulant products and of which the wall 29 is constituted by a membrane of elastically deformable material traversed by a multiplicity of pores, allowing the anti-coagulant products located inside said pouch to exsude outwardly and be mixed with the blood being stored, said pouch of elastically deformable material being called upon to take an expanded position (with opening of the pores and passage of anti-coagulant products) in the phase of depression of the storage vessel.

10. Device according to any one of Claims 1 to 9, characterized in that the base of the collection vessel 25 communicates by a passage 27 with an evacuation chamber 70 containing second filtration means formed by a filtering cloth in the form of a cone, the apex being oriented downwardly and terminating in an outlet 71a to which are connected transfusion means.

11. Device according to any one of Claims 1 to 10, characterized in that it comprises a tank 2 constituting the filtration vessel in upper position and fast with a second tank 18 internally containing the collection vessel 25, the two superposed tanks being shaped in the form of coaxial bodies of revolution whose walls extend substantially, being fast with one another.

12. Device according to Claim 11, characterized in that the upper tank 2 and the lower tank 18 are made of transparent, haemocompatible, synthetic material.

**Patentansprüche**

1. Vorrichtung für die Entnahme und Re-Injektion von eigenem Blut in den Organismus des Patienten, um eine Blutung zu kompensieren, bestehend aus:

a) Vorrichtungen zur Entnahme von Blut durch Ansaugen, die in unmittelbarer Nähe der Rißstelle der Gefäße angebracht werden können,

b) einem ersten Filterbehälter (2) zum Auffangen des von der Entnahmevorrichtung stammenden Blutes, wobei dieser Filterbehälter (2) mit Filterlagen (9, 9a, 9b, 9c) versehen ist,

c) einem zweiten sich unterhalb des Filterbehälters befindenden Sammel- und Speicherbehälter (25) zum Auffangen des Blutes, das durch die Schwerkraft sowie durch Unterdruck vom Filterbehälter (2) abfließt, wobei der Boden des Sammel- und Speicherbehälters (25) durch ein unterhalb gelegenes Ausgangsrohr (71a) mit Vorrichtungen zur Rücktransfusion in den Blutkreislauf des Patienten verbunden ist, wobei

d) der vorgenannte Sammel- und Speicherbehälter am oberen Teil einerseits mit einer Unterdruck-Quelle (39a), andererseits mit dem Filterbehälter (2) in Verbindung steht, wobei der Unterdruck innerhalb des Sammelbehälters erzeugt wird und am Filterbehälter anliegt, wodurch das Ansaugen und Filtrieren des Bluts unter teilweisem Vakuum sowie das Abfließen des Bluts aus dem Filterbehälter (2) zum Sammel- und Speicherbehälter (25) bewirkt wird, wobei

e) der vorgenannte Sammel- und Speicherbehälter (25) überdies an seinem oberen Teil mit einer Überdruck-Quelle (43) in Verbindung steht, die durch Überdruck in der gasförmigen Phase des vorgenannten Behälters (25) die Blutzirkulation in Richtung auf die Vorrichtung zur Reinjektion durch Transfusion in das Blutsystem des Patienten bewirkt,

dadurch gekennzeichnet, daß

f) der Filterbehälter (2) in seinem oberen Teil eine mit dem vorgenannten Filterbehälter in Verbindung stehende Quelle (15) mit anti-koagulierenden Produkten aufweist, die die nebelartige Verteilung dieser anti-koagulierenden Produkte bewirkt, um die gasförmige Phase des Filterbehälters oberhalb und stromaufwärts der in diesem Behälter vorgesehenen Filtervorrichtungen (2) zu füllen.

g) daß der Sammel- und Speicherbehälter (25) einen zweiten Behälter (30) mit anti-koagulierenden Wirkstoffen umfaßt, welcher von dem aufgefangenen Blut bedeckt wird, und der Dispersionsmittel enthält, die die Verteilung dieser Wirkstoffe in das aufgefangene Blut bewirken,

h) daß die Verbindung zwischen den beiden Behältern, nämlich dem Filterbehälter (2) und dem Sammel- und Speicherbehälter (25) einerseits untereinander und andererseits mit den Unterdruck- (39a) und Überdruck-Quellen (43), von einem Schieber mit mehreren Ausgängen (34, 48) bestimmt wird, wobei der Schieber in einer ersten Position den Sammel- und Speicherbehälter (25) von der Überdruck-Quelle trennt, während dieser Behälter mit der Unterdruck-Quelle und dem Filterbehälter in Verbindung gebracht wird, wodurch das Blut in Richtung des Filterbehälters und weiter zum Sammel- und Speicherbehälter angesaugt wird, und der Schieber (34, 48) in einer zweiten Position den Sammel- und Speicherbehälter (25) von der Überdruck-Quelle (39a) und von dem Filterbehälter trennt, indem der Sammel- und Speicherbehälter (25) mit der Überdruck-Quelle in Verbindung gebracht wird, wodurch der so erzeugte Überdruck in der Höhe des Sammel- und Speicherbehälters die Verdrängung des Bluts in Richtung der Vorrichtungen zur Rück-Transfusion bewirkt.

2. Vorrichtung nach Anspruch 1, dadurch gekennzeichnet daß der Sammel- und Speicherbehälter (25) aus einer Tasche aus weichem Kunststoff besteht, der sich zum Aufnehmen des Blutes aus dem Filterbehälter eignet, wobei die Tasche durch den Schieber (33, 34) in ihrem oberen Teil mit dem Filterbehälter (2) verbunden ist, und in ihrem unteren Teil mit einer Abführleitung in Verbindung steht, die zweite Filtervorrichtungen (68) aufweist, wobei der Auffangbehälter (25) innerhalb einer steifen Wanne (18) angebracht ist, und der Raum zwischen der Wand der Wanne und dem Behälter oder der Tasche (25) einen Luftmantel (61) bildet, der durch einen aus einem Entlüftungsrohr bestehenden Durchgang mit der Tasche (25) verbunden wird, und an seinem oberen Teil und, je nach Stellung des mehrere Ausgänge (33, 34) aufweisenden Schiebers, abwechselnd mit der Unterdruck-Quelle oder der Überdruck-Quelle in Verbindung bringbar ist.

3. Vorrichtung nach einem der Ansprüche 1 und 2, dadurch gekennzeichnet, daß die Filterwanne (2) im wesentlichen kegelstumpfförmig ist, wobei die kleine Basis unten ist und einen trichterförmigen Boden aufweist, dessen Mittelteil (6) mit dem Verbindungsdurchgang zwischen der Filterwanne (2) und dem Sammelbehälter (25) versehen ist, wobei die Filterwanne von einem mit Henkeln (65) versehenen Deckel (3) bedeckt wird, um die gesamte Vorrichtung aufzuhängen, und die Filterwanne innen eine Vielzahl von übereinanderliegenden Filtergeweben (9, 9a, 9b, 9c) mit von oben nach unten abnehmender Maschenweite aufweist.

4. Vorrichtung nach Anspruch 3, dadurch gekennzeichnet, daß jedes dieser Filtergewebe von einem umgebenden Ring (10, 10a, 10b, 10c) gestützt wird, deren Umfänge vom oberen Filter zum unteren Filter abnehmen, was ein Anliegen der mehreren Ringe an der kegelstumpfförmigen Wand in der übereinandergestapelten Lage bewirkt.

5. Vorrichtung nach einem der Ansprüche 1 bis 4, dadurch gekennzeichnet, daß die Filtergewebe eine konische Form aufweisen, wobei die Spitze oben liegt.

6. Vorrichtung nach einem der Ansprüche 3 bis 5, dadurch gekennzeichnet, daß das oberste Filtergewebe (9) in seinem Mittelteil eine konische Form aufweist, wobei die Spitze oben liegt und konzentrisch zu den unterhalb liegenden Filtergeweben (9a, 9b, 9c) angeordnet ist, und der konische Mittelteil des oberen Gewebes (9) an einen Umfangsteil eines umgekehrten Kegelstumpfes angepaßt ist, wobei dieses oben liegende Filtergewebe vertikale Zähnchen (11) aufweist, die in dem Blut des Patienten sich eventuell befindliche organische Überreste und Blutklümpchen zurückhalten und stillegen.

7. Vorrichtung nach einem der Ansprüche 1 bis 6, dadurch gekennzeichnet, daß der Deckel (3) der Filterwanne (2) einen an seinem oberen Teil mit einer abnehmbaren Verschlußkappe (16) aus luftdichtem, jedoch weichem Material, z.B. Elastomer, vorgesehenen Balg (15) aufweist, der mit Hilfe einer Injektionsspritze und einer Nadel, die durch diese Verschlußkappe geführt wird, mit anti-koagulierenden Wirkstoffen gefüllt werden kann, wobei durch den Balg (15) die anti-koagulierenden Wirkstoffe in den oberen Teil der Filterwanne (2) nebelartig verteilt werden.

8. Vorrichtung nach einem der Ansprüche 1 bis 7, dadurch gekennzeichnet, daß die Filterwanne (2) an die sich darunter befindliche Wanne (18) angepaßt wird, die den Sammel- und Speicherbehälter über einen ringförmigen Gürtel (33) enthält, der an zwei diametral gegenüberliegenden Stellen von einem rohrförmigen Kanal durchdrungen ist, wodurch die konische Öffnung (34) entsteht, die mit einem Küken (48) des mit mehreren Ausgängen versehenen Schiebers besteht, wobei das Küken durch einen außerhalb liegenden Bedienungshebel (47) gedreht werden kann, und das Küken zwei Kanäle (49) und (50) aufweist, um gleichzeitig den Filterbehälter (2) durch die koaxialen, mit jedem der zu beiden Behältern gehörenden Durchgängen (6) und (26) in Verbindung zu setzen, wobei der Durchgang (50) gleichzeitig die Verbindung des Sammelbehälters (25) mit der Unterdruck-Quelle (39a) ermöglicht (wobei das Ansaugen bewirkt wird), während durch eine zweite Stellung des Bedienungshebels (47) und des Kükens (48) der Filterbehälter vom Sammelbehälter getrennt wird.

9. Vorrichtung nach einem der Ansprüche 1 bis 8, dadurch gekennzeichnet, daß die den Sammel- und Speicherbehälter bildende Tasche (25) im Innern einen mit anti-koagulierenden Wirkstoffen gefüllten Beutel enthält, dessen Wand (29) aus einer Membran aus elastisch verformbarem Material besteht, die eine Vielzahl von Poren aufweist, die ermöglichen, daß die sich in dem Beutel befindlichen anti-koagulierenden Wirkstoffe nach außen dringen, und sich mit dem den Sammel- und Speicherbehälter durchströmenden Blut vermischen, wobei der aus elastisch verformbarem Material bestehende Beutel sich ausdehnt (wobei sich die Poren öffnen und die anti-koagulierenden Wirkstoffe austreten), wenn der Auffangbehälter einem Unterdruck ausgesetzt wird.

10. Vorrichtung nach einem der Ansprüche 1 bis 9,
dadurch gekennzeichnet, daß der Boden des Sammelbehälters (25) über einen Durchgang (27) mit einer Ausscheidungskammer (70) verbunden wird, die zweite aus Filtergeweben bestehende Filtermittel mit konischer, die Spitze nach unten gerichteter Form enthält, die in einem Ausgang (71a) mündet, an den Vorrichtungen zur Transfusion anschließbar sind.

11. Vorrichtung nach einem der Ansprüche 1 bis 10, dadurch gekennzeichnet, daß sie eine Wanne (2) aufweist, die die sich darüber befindliche, mit einer zweiten Wanne (25) verbundene Filtrationswanne bildet, wobei die zwei übereinander angeordneten Wannen in ihren koaxialen Drehkörpern einander angepaßt sind, und die Wände ineinander übergehen und miteinander verbunden sind.

12. Vorrichtung nach Anspruch 11, dadurch gekennzeichnet, daß die obere Wanne (2) und die darunter liegendende Wanne (18) aus transparentem und blutverträglichem Kunststoff bestehen.

FIG.1

FIG. 3

FIG. 2

18

# FIG. 4

FIG. 5

FIG.6

FIG.14

FIG.15

20

FIG.7

FIG. 8

FIG. 9

FIG.10

FIG.11

FIG.12

FIG.13